# EUROPEAN PATENT SPECIFICATION

(11) **EP 1 175 617 B1**
(45) Date of publication and mention of the grant of the patent: **14.09.2005**
(21) Application number: 00927939.9
(22) Date of filing: 26.04.2000
(51) Int. Cl.: G01N 33/566, G01N 30/46

(54) **IDENTIFICATION OF LIGANDS FOR ORPHAN RECEPTORS USING MASS SPECTROMETRY**
IDENTIFIZIERUNG VON LIGANDEN FÜR "ORPHAN"-REZEPTOREN UNTER VERWENDUNG VON MASSENSPEKTROMETRIE
IDENTIFICATION DE LIGANDS POUR DES RECEPTEURS ORPHELINS PAR SPECTROMETRIE DE MASSE

(30) Priority: 26.04.1999 EP 99201301
(43) Date of publication of application: 30.01.2002
(73) Proprietor: Screen TEC B.V., 2333 CC Leiden (NL)
(72) Inventor: VAN DER GREEF, Jan, NL-3971 BM Driebergen (NL); IRTH, Hubertus, NL-1078 NP Amsterdam (NL)
(74) Representative: Prins, Adrianus Willem
(86) International application number: PCT/NL2000/000268
(87) International publication number: WO 2000/065353

(56) References cited:
- WO-A-96/37777
- WO-A-97/01755
- WO-A-97/43301
- WO-A-98/56028
- NEDVED, MICHAEL L.; HABIBI-GOUDARZI, SOHRAB; GANEM, BRUCE; HENION,: "Characterization of Benzodiazepine "Combinatorial" Chemical Libraries by Online Immunoaffinity Extraction, Coupled Column HPLC-Ion Spray Mass Spectrometry-Tandem Mass Spectrometry" ANALYTICAL CHEMISTRY, vol. 68, no. 23, 1 December 1996 (1996-12-01), pages 4226-4236, XP002117165 cited in the application
- SIEGEL, MARSHALL M.; TABEI, KEIKO; BEBERNITZ, GERALDINE A.; BAUM, ELLEN Z.: "Rapid methods for screening low molecular mass compounds non-covalently bound to proteins using size exclusion and mass spectrometry applied to inhibitors of human cytomegalovirus protease" JOURNAL OF MASS SPECTROMETRY, vol. 33, no. 3, 1998, pages 264-273, XP002117166 cited in the application
- DUNAYEVSKIY, YURIY M.; LAI, JAN-JI; QUINN, CHERYL; TALLEY, FRANK; VOUROS, PAUL: "Mass spectrometric identification of ligands selected from combinatorial libraries using gel filtration" RAPID COMMUNICATIONS IN MASS SPECTROMETRY, vol. 11, no. 11, 1997, pages 1178-1184, XP002117167 cited in the application
- BLOM, KARL F.; LARSEN, BARBARA S.; MCEWEN, CHARLES N.: "Determining Affinity-Selected Ligands and Estimating Binding Affinities by Online Size Exclusion Chromatography/Liquid Chromatography-Mass Spectrometry" JOURNAL OF COMBINATORIAL CHEMISTRY, vol. 1, no. 1, January 1999 (1999-01), pages 82-90, XP002117168 cited in the application
- HUYER, GREGORY; KELLY, JOHN; MOFFAT, JASON; ZAMBONI, ROBERT; JIA, ZONGCHAO; GRESSER, MICHAEL J.; RAMACHANDRAN, CHIDAMBARAM: "Affinity selection from peptide libraries to determine substrate specificity of protein tyrosine phosphatases" ANALYTICAL BIOCHEMISTRY, vol. 258, no. 1, 1998, pages 19-30, XP002117169 cited in the application
- HSIEH YF, GORDON N, REGNIER F, AFEYAN N, MARTIN SA, VELLA GJ: "Multidimensional chromatography coupled with mass spectrometry for target-based screening" MOLECULAR DIVERSITY, vol. 2, no. 4, April 1997 (1997-04), pages 189-196, XP002117170 cited in the application
- KAUR S, MCGUIRE L, TANG D, DOLLINGER G, HUEBNER V: "Affinity selection and mass spectrometry-based strategies to identify lead compounds in combinatorial libraries" JOURNAL OF PROTEIN CHEMISTRY, vol. 16, no. 5, July 1997 (1997-07), pages 505-511, XP002117171 cited in the application

## Description

The present invention relates to the on-line coupling of mass spectrometry (MS) to continuous-flow separation techniques for detecting 'orphan' analytes, *viz*. affinity molecules which do not have known ligands, such as orphan affinity proteins, *e*.*g*. orphan receptors. In a further embodiment, this on-line detection method can be used as a screening method for assays with an unknown ligand. Furthermore, this invention relates to compounds detected by this method and the use of these compounds as a ligand for affinity molecules.

A range of biochemical assays based on continuous flow biochemical detection has been developed during the recent years. Typically, the affinity interaction between ligand and affinity protein has been monitored by measuring the concentration of highly sensitive, usually fluorescence labeled reporter molecules using either heterogeneous or homogeneous biochemical detection strategies. When dealing with 'orphan' receptors however, *i*.*e*. receptors for which 'affinity' ligands have not been found so far, new strategies have to be developed, which enable so-called 'label-free detection' of affinity ligands. In contrast to the label-based assays, which usually allow the non-binding analytes from the sample to co-elute with the reporter molecules, label-free assays for orphan receptors demand strict separation of both binding and non-binding ligands.

By configuring restricted access columns and hollow fiber modules in the biochemical part of the system, affinity ligands can be isolated from complex biological matrices. Subsequently, coupling the biochemical setup to mass spectrometry allows the on-line detection of ligands exhibiting affinity for the orphan affinity molecule.

It is known from the prior art that biochemical assays are highly sensitive detection techniques, which combine the selectivity of biospecific interactions with the sensitive detection of labels used as reporter molecules.

An example of such a technique is described in WO 91/13354. This document describes a flow immunosensor wherein an antibody specific to an affinity molecule is immobilized on a support. The antibody's binding sites are saturated with a labeled form of a known ligand. A liquid to be analyzed is contacted with the antibody to allow any affinity molecule present in the liquid to displace the labeled antigen. Finally, the displaced labeled antigen is detected.

However, it is known as well that biochemical assays suffer from the problem of cross-reactivity, which means that antibodies react with more than one analyte. This leads to erroneous results. For this reason, biochemical assays are frequently combined with a fractionation step, e.g., a separation step using HPLC or another type of liquid chromatography.

In the field to which the present invention relates, there is a demand for on-line coupling of a fractionation step and an biochemical assay detection system. Several approaches have been proposed and described to perform continuous-flow biochemical assays. Most of these biochemical assays are in the form of a postcolumn reaction detection system based on a sequential addition type biochemical assay.

Cassidy *et al*., Anal. Chem. 64 (1992), 1973-1977 disclose a kinetically controlled immunoassay based on the sequential addition of antibody, sample and label on a protein A column, performing immunoassays for albumin and transferrin in less than 1 minute.

Nilson *et al*., J. Chromatography, 597 (1992), 383-389 describe a continuous-flow competitive assay involving enzyme-labeled antibodies. The system described was coupled to a size-exclusion column to allow the monitoring of activity.

These prior art techniques which are of a sequential nature are not suitable for on-line coupling to liquid chromatographic or other fractionation systems, since these techniques do not allow the continuous monitoring of the fractionation effluent.

In an article in Analytical Chemistry 68 (1996), 4226-4236, Nedved *et al*. describe a method to characterize benzodiazepines. The method encompasses the binding of benzodiazepines to antibodies on a protein-6-column. The complexes formed are eluted using a pH-step. The benzodiazepines are separated from the antibodies by using a restricted access (RA) column, leaving the benzodiazepines in the column material. After this three step method that cannot be used in a continuous process, the benzodiazepines are eluted from the RA column and transported to a mass spectrometer for detection.

Siegel *et al*. Describe in J. Mass Spectr. 33 (1998), 264-273 a method wherein a sample containing protease and inhibitors is incubated for 1 hour and subsequently the sample is subjected to a separation on molecular weight. This separation step can be carried out using a gel-permeation chromatography technique or a microconcentration technique. The method is not suitable for a continuous detection method.

Neither suitable for an continuous on-line detection technique is the method described by Dunayevskiy *et al*. in Rapid Communications in Mass Spectrometry 11 (1997), 1178-1184. The method requires a gel-filtration column to encapture unbound components and to pass proteinaceous material.

Blom *et al*. described in J. Combinatorial Chem. 1 (1999), 82-90 a method wherein a mixture of protein/ligand complexes and unbound ligand are separated or a size exclusion column, wherein the complexes are caught in a protein trap. From this trap the complexes are eluted by means of a gradient LC pump. The effluent is led to a MS.

Neither a suitable continuous on-line method is described by Huyer *et al*. in Analytical Biochemistry 258 (1998), 19-30 involving separation of peptide-enzyme complexes from unbound enzymes using gel-filtration spin columns, followed by on-line concentration using a C₁₈-cartridge and injection in a MS.

In the article of Irth *et al*. in the Journal of Chromatography 633 (1993), 65-72 and in the articles of Oosterkamp *et al*. in Anal. Chem. 66 (1994), 4295-4301, and in the Journal of Chromatography 653 (1994), 55-61, a method for the on-line detection of digoxigenin and its metabolites is described. The on-line detection process comprises the direct injection of a sample containing digoxigenin and its metabolites, a liquid chromatographic (LC) fractional separation step, the mixing of the effluent of the LC column with fluorescein-labeled antibodies against digoxigenin, the removal of free labeled antibodies from the mixture via passage through a small column packed with an antigen-bound support, and detection of the strongly fluorescent biochemical complexes.

The digoxigenin system described is based on association reactions of antibodies and antigens eluting from the analytical column. By the use of fluorescein-labeled antibodies detection limits in the nanomolar range are obtained.

This prior art assay is a heterogeneous detection system. It requires a separation step between free and bound label. Examples for free/bound label separation techniques are restricted-access phases and hollow-fibre modules.

The biochemical reagent in the previously described prior art assay consists of fluorescein-labeled fragments of anti-digoxigenin antibodies which were immunopurified and are commercially available. The commercial availability of purified, labeled antibodies is however exceptional. In almost all cases, antibodies are only available in unlabelled state in crudely purified antiserum. Although labeling and purification schemes for antibodies (or other affinity proteins) are known to the person skilled in the art, it will be preferred to use antisera, which may be commercially available, without any pretreatment.

Hsieh *et al*. (Molecular Diversity, 2 (1996), 189-196), describe a screening method using chromatography coupled with mass spectrometry. Their method requires a dual run, first in the absence of a library of protein targets and then with the library. This means that background compounds, especially the affinity proteins causing severe background signals over a broad range in the mass spectrum, are always present in this assay type, which limits the efficiency, selectivity and flexibility of the method considerably.

Kaur *et al*. (Journal of Protein Chemistry 16, No 5 (1997)), describe a method in which active components in a combinatorial library are bound to a receptor, isolated by size exclusion chromatography and trapped on a column after which the desorption solvent is used to dissociate the affinity protein/ligand complex. Subsequent identification is done by mass spectrometry, but with a background of the affinity protein.

The methodology described by Hsieh and Kaur is thus hampered by severe background signals in the mass spectrometer of the present affinity protein.

One of the advantages of the present invention is that it provides the possibility to detect the unknown ligand in the absence of a protein background in the MS. Especially for low concentration this is a requirement for successful assays.

One aim of the invention is to provide an improvement on the known assays by providing a technique, which does not require the use of known ligands.

The above mentioned problems are overcome by the provision of an on-line detection method comprising the on-line coupling of the effluent of a fractionation step to a mass spectrometer, which effluent possibly contains analytes, which method comprises the addition of a controlled amount of an affinity molecule, such as an affinity protein, to said effluent, whereby the affinity molecules bind analytes in the effluent, followed by a separation step using a restricted-access support, whereby the analyte-affinity molecule complex is permeated while smaller molecules are retained in the restricted access column, followed by a suitable dissociation step to dissociate the analyte-affinity molecule complex, followed by a second separation step in which the dissociated analyte and affinity molecules are separated, followed by detection of the analyte using the mass spectrometer.

The use of the first restricted access column provides the permeation of the analyte-affinity molecule complex while the smaller molecules are retained.

The dissociation step results in the dissociation of the complex. By subsequently separating the affinity molecules from the effluent the analyte can be directed to the MS.

In particular, a suitable affinity molecule for analytes to be detected - which affinity molecule may e.g. be an affinity protein - is added to the effluent of a liquid chromatography or a capillary electrophoresis column to react with the analytes eluting from the column.

The second separation step is carried out either by using a restricted-access support or by using a hollow fiber support.

When using a restricted-access support in the second separation step the affinity molecule is retained, followed by elution of the affinity molecule from the restricted-access support using a suitable carrier stream, and directing the eluted stream to the mass spectrometer. Prior to elution of the affinity molecule, a washing step can be carried out to remove the dissociation solvent and the receptor proteins from the restricted-access column. In order to prevent false positives, it is important that the conditions, with respect to concentrations of compounds such as solvents, of the second restricted-access column are substantially constant and substantially the same as in the first restricted-access column.

When using a hollow fiber support in the second separation step the ligand is permeated and the permeate is directed to the mass spectrometer.

Preferably the dissociation step is a low pH shock, such as injection of a hydrochloric acid solution with a pH below 2.5, contacting with a high ionic strength solution, contacting with an organic solvent and/or contacting with a chaotropic reagent.

The fractionation step according to the present invention can be a liquid chromatography separation, a capillary electrophoresis step or a combinatorial chemistry system, which is optionally followed by a separation step which removes the high molecular weight background.

Preferred liquid chromatography fractionation steps comprise HPLC, reversed phase HPLC, capillary electrophoresis (CE), capillary electrochromatography (CEC), isoelectric focusing (IEF) or micellar electrokinetic chromatography (MEKC), all of which techniques are known to the person skilled in the art.

All known MS options and techniques can be used. Preferably the mass spectrometer is of the type chosen from the group consisting of electrospray ionization type, atmospheric pressure ionization type, quadrupole type, magnetic sector type, time-off-flight type, MS/MS, MSⁿ, FTMS type, ion trap type and combinations thereof.

The present invention enables the detection of ligands for orphan receptors.

The combination of the fractionation step with the MS detection step in accordance with the present invention greatly enhances the performance of both techniques. The combined techniques provide an analytical method, which is characterized by a high selectivity and a high sensitivity. Further, the problem associated with cross-reactivity does not occur when using the method of the present invention.

The method of the present invention uses bioaffinity molecules such as receptors, to detect any compounds showing high affinity for the ligand binding site of said affinity molecule. The compounds to be detected may be biochemical compounds but are not in any way restricted thereto.

A system of high-throughput screening to be used in drug discovery may, for instance, comprise the following steps. Complex samples generated for instance by an upstream combinational chemistry system are prefractionated in fractions containing compounds of similar polarity using, *e.g.*, a solid-phase extraction technique or electrophoretic sample handling principles. Each fraction may additionally be fractionated using, *e.g.*, either analytical or preparative-scale liquid chromatographic fractionating columns. The compounds eluting from said LC column are on-line detected using a suitable affinity molecule detection technique. Where preparative-scale fractionating columns are applied, a post-column flow-split will be made. One of the two flow streams is subjected to detection using the affinity molecule detection technique; the other stream is directed to a fraction collector. Dependent on the signal obtained from the affinity molecule detector, fractions containing compounds causing a positive response will be collected while fractions causing a negative response will be discarded. This complete screening method can be automated using known valve-switching processes.

A suitable fractionation method to be used in the methods of the present invention comprises a liquid chromatography fractionation or a capillary electrophoresis step. Other fractionation techniques which are known to the person skilled in the art and which allow a relatively continuous output stream can, however, be used as well.

In a preferred embodiment, the liquid chromatography separation step is a reversed phase HPLC step.

It will be understood that according to the present invention as the effluent of a fractionation step is also to be understood the effluent of a flow injection, in which a mixture of different compounds is injected, optionally followed by a trapping step, for example using a restricted-access column. The separation step of the method according to the present invention then fulfills the fractionation, since the compounds of interest are fractionated by the separation step. The required selectivity can be obtained by operating the MS selectively tracing of the MS.

Since the flow injection technique provides a flexible and fast screening method, it is a preferred embodiment of the present invention.

All modes of MS-operation are possible in Flow Injection mode, however, due to the higher background, especially the very selective modes which comprise detecting ions of a selected single m/z trace or of selected multiple m/z traces are suitable.

Restricted-access supports are well-known to the person skilled in the art. In general, restricted-access supports, such as C₁₈-silica (for example Lichrospher™ C18 ADS from Merck) packed columns, are capable of retaining smaller sized molecules while permeating the larger molecules. The type of restricted-access supports and the conditions under which they are operated, will depend on the type of assay. A person skilled in the art will be able to perform routine experiments to establish the optimal conditions and materials for each case.

Hollow fiber materials, for example made of polysulphon (obtainable from Amicon, Ireland) are well-known to the person skilled in the art. In general, hollow fiber modules are capable of permeating smaller sized molecules while retaining the larger molecules. Also the type of hollow fiber supports and the conditions under which they are operated, will depend on the type of assay. Again, the person skilled in the art will be able to perform routine experiments to establish the optimal conditions and materials for each case.

As already mentioned, the fractionation step can preferably be a liquid chromatography separation or a capillary electrophoresis step. It has been found that it is very advantageous when this fractionation step is followed by a separation step, for example by using a hollow-fiber module, since this removes the high molecular weight background. Preferred liquid chromatography fractionation steps comprise HPLC, reversed phase HPLC, CE, CEC, IEF or MEKC, all of which techniques are known to the person skilled in the art.

All possible variations in MS techniques known in the art can be profited from according to the present invention. preferably, the MS is of the type chosen from the group consisting of electrospray ionization type, atmospheric pressure ionization type, quadrupole type, triple quadrupoles (QQQ) type, magnetic sector type, time-off-flight type, MS/MS, MSⁿ, FTMS type, ion trap type and combinations thereof.

For example, in scanning mode to trace compounds, low resolution MS with all possible instrumental designs of MS can be used, in particular quadrupole, magnetic sector, time-off-flight, FTMS and ion-trap. This generates typically molecular weight data with nominal mass accuracy.

When high resolution MS is applied, using all possible high resolution instrumental designs, in particular magnetic sector, time-off-flight, FTMS and ion trap, molecular weight data with high mass accuracy combined with the elemental composition of the compound can be obtained.

Other known MS techniques comprise tandem MS, such as MS/MS or MSⁿ (for example MS³). Application of these techniques enables the collection of structural information of the ligands, which is a preferred embodiment of the present invention. The data in scanning mode can be acquired in data-dependent mode which means that for each peak observed automatically the tandem MS measurement is performed. In addition to this the fragmentation induced in the tandem MS measurement can be coupled by more sophisticated procedures, for example a broad band excitation, which also fragments expected fragments of less importance, such as the [protonated molecule-H₂O]⁺ peak in natural product screening.

The MS can also be operated in single/multiple ion monitoring mode, which can be used for highly selective detection.

Single/multiple ion monitoring mode can be used for highly selective detection. Using low resolution MS in single/multiple ion monitoring mode with all possible low resolution instrumental designs, in particular quadrupole, magnetic sector, time-off-flight, FTMS and ion trap, selective detection based on monitoring of previously determined m/z trace can be obtained. When high resolution MS is applied all possible instrumental designs of MS can be used, especially quadrupole, magnetic sensor, time-off-flight, FTMS and ion-trap. This enables typically very selective detection based on monitoring in high resolution previously determined m/z trace. When tandem MS is applied, all possible instrumental designs, in particular ion trap, quadrupole-time-off-flight (Q-TOF), FTMS and combinations of sector instruments with quadrupoles and ion traps, can be used. This enables the very selective detection based on monitoring a resulting peak or peaks in MS/MS and/or MSⁿ measurements.

The assays according to the present invention can be based on continuous monitoring of the HPLC effluent after a separation step, for instance with a hollow fiber step, to remove the high molecular weight background. The MS is typically set to operate in one of single/multiple ions mode options.

With the assays according to the present invention the tracing of (bio-)active compounds in solutions of complex nature, for instance biological fluids or extracts, natural product extracts, solutions or extracts from biotechnological processes, resulting from chemical experiments, such as combinatorial technologies and processes and the like can be performed with a higher efficiency, selectivity and flexibility. Moreover, the present invention provides the possibility to limit the disturbance of background compounds.

The present invention further enables the identification of compounds based on conventional mass spectra, high resolution data or MSⁿ based spectra.

With the method of the present invention it is also possible to perform library searching, based on a variety of mass spectrometric experiments enabling the screening of large series of samples and classifying these in classes based on similar active compounds, without the need for full identification of the compounds.

The assay method of the present invention can be applied in miniaturized formats of assay-based systems, for instance with chip technology based screening systems.

In the methods of the present invention all molecules capable of interaction with or binding to other molecules can be used as affinity molecule.

On-line coupling as used in the methods of the present invention requires fast reaction times in order to minimize extra-column band broadening. This means that affinity molecule-ligand interactions having reaction times in the order of minutes rather than hours should be considered. The suitable binding conditions under which the affinity molecules bind to the analyte comprise a contact time, which is in the same order of magnitude. Suitable binding conditions are conditions that provide optimal binding between the affinity molecules and the analyte. It will be understood that the precise conditions, such as temperature, residence time, chemical composition, will depend strongly on the type of assay and the proteins used therein.

The present invention will be described in further detail while referring to the following examples.

### Example 1 Restricted-access separation

Phosphate buffered saline (PBS) carrier phase solution, affinity protein solution, dissociation solution and desorption solution were pumped by Knauer ministar pumps. The flushing solution was added using a Minipuls 3 peristaltic pump. Two Rheodyne sixport valves were used to enable flushing and column switching. A third rheodyne injection valve was used to perform the loop injections (5 µl injection volume). The carrier phase consisted of PBS buffer (pH 7.0) which was pumped at a flow rate of 40 µl/min. A solution of anti digoxigenin Fab fragments (5 µM) in PBS was added to carrier phase at 80 µl/min. During control experiments the anti digoxin Fab fragments were switched with either PBS or PBS solutions containing 2 µM of goat antibiotin. The dissociation solution consisted of 1 M hydrochloric acid which was added at 260 µl/min. The desorption buffer consisted of 80% MeOH and 2 mM ammonium acetate. The rinsing solution was composed of 5% isopropanol/2 mM ammonium acetate and was used to flush the second RA-column for one minute after trapping at 300 µl/min. The dimensions of the first and second restricted access columns equaled 4 x 15mm and 2 x 10 mm respectively. The RA-material used was Lichrospher C18 ADS (Merck). Reactions were carried out in knitted open tubular Teflon reaction coils (0.5 mm ID), using reaction times of 30 s and 30 s for the first and second biochemical reaction respectively. Detection was carried out by a Finnigan LCQ mass spectrometer.

After injection in the carrier phase, digoxin was allowed to react for 30 s with anti digoxigenin Fab fragments. After reaction Fab bound digoxin was able to pass the first restricted access column, whereas excess unbound molecules were trapped onto the first RA-column. After passing the first RA-column the affinity complex was dissociated by a pH shock using a solution of hydrochloric acid. Again the mixture was allowed to react for 30 s after which the ligand could be trapped onto the second RA-column. Three minutes after injection, the second RA-column was flushed for one minute, after which the second RA-column was switched into the desorption solution. Digoxin was desorbed and subsequently detected by the MS.

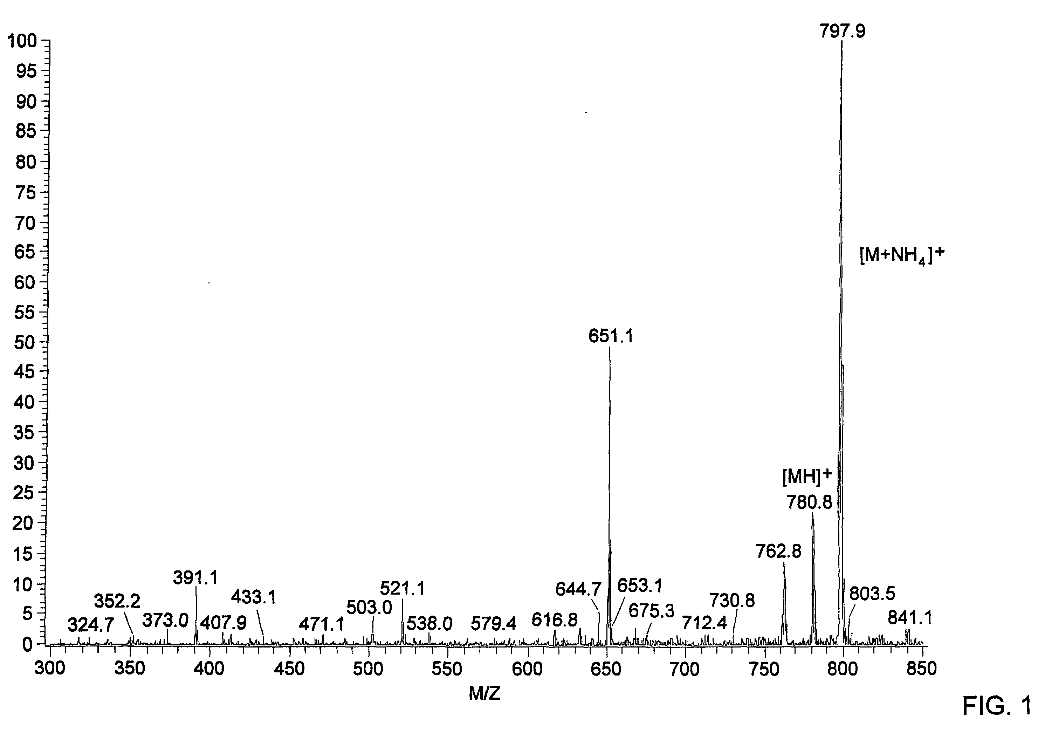

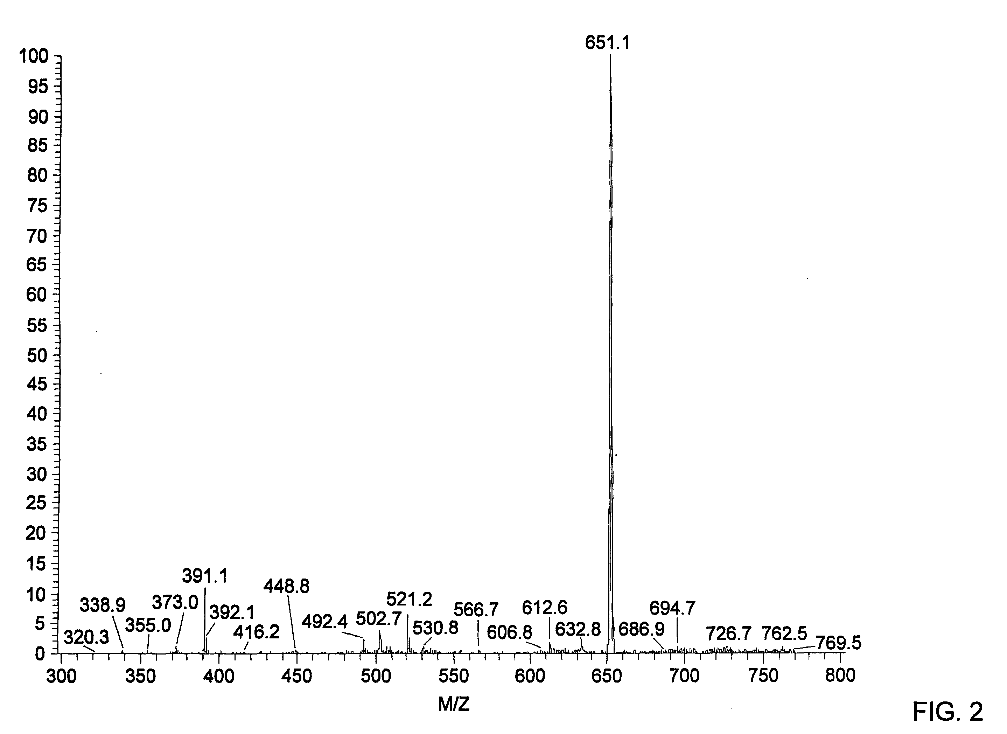

Figure 1 presents a typical MS spectrum of digoxin. Apart from the protonated molecule (780.8), an ammonium cluster can be observed at 797.9, whereas the m/z ratios of 762.8 and 651.1 represent the loss of respectively water and a sugar group. Wideband activation of the protonated molecule (780.8 +/- 18) at 45% collision energy fragmentates both the parent mass as well as the ammonium complex to 651.1, which can be observed as the base peak in MS/MS spectra under the conditions used (figure 2). During assay operation digoxin was detected using both MS and MS/MS (m/z 797.9 and 651.1 respectively).

Injecting 5, 50 and 500 ng digoxin in the assay system with and without anti digoxigenin Fab fragments showed that digoxin was completely trapped when the affinity protein was absent. However addition of anti digoxigenin Fab fragments showed increasing response of digoxin (measured at m/z 651.1), demonstrating the ability of the system to detect digoxin using the MS-based orphan assay.

In order to check the robustness of the ms-bcd system, the anti-digoxigenin Fab fragment solution was replaced by goat antibiotin. Injection of 0, 5, 50 and 500 ng digoxin showed that the signal obtained for 500 ng is still comparable to the blank, meaning that the excess of digoxin is adequately trapped on the first restricted access column.

### Example 2 Hollow fiber separation

The set-up consisted of two Pharmacia (Uppsala, Sweden) P3500 pumps which delivered the mobile phase and affinity protein solution, one Gilson 306 LC-pump which delivered an acidic solution and two Gilson Minipuls 3 peristaltic pumps which respectively created backpressure at the retentate outlet and added phosphate buffer to the permeate phase. Mobile phase, affinity protein solution and acidic solution were pumped at a flow-rate of 0.2 ml/min. Acidic conditions in the permeate phase were neutralized by the addition of a phosphate buffer (100 µl/min). Injections were performed by an Aspec autosampler equipped with two Rheodyne six port valves (injection volume 20 µl). A Finnigan LCQ mass spectrometer was used for detection.

0.15 ml/min PBS carrier (136.9 mmol/l NaCl, 2.7 mmol/l KCl, 9.2 mmol/l Na₂HPO₄ and 1.8 mmol/l K₂PO₄) comprising eighty randomly chosen drugs, each in the 1 µM range, was mixed with 10 nM goat antibiotin (0.15 ml/min, obtained from Pierce, Rockford, Illinois, USA) by means of an inverted Y-piece. The mixture is allowed to react for 48 seconds in a reaction coil prior to the separation of unbound ligands and affinity complexes by a restricted-access column (C18 alkyl diol, Merck Lichrospher™ ADS, 15 x 4 mm). Subsequently after passing the restricted access column the pH of the mobile phase is lowered to 2 by addition of hydrochloric acid (0.3 ml/min), causing the affinity complex to dissociate in a reaction coil within 10 s reaction time. On-line filtration by means of a hollow fiber module (polysulphon, obtained from Amicon, 0.5 mm I.D., 30 kDa cut-off, Module I.D. was 0.85 mm) then separates affinity molecules from ligands. The ligands could all subsequently be detected with the MS.

### Example 3 Detection of digoxin and related compounds in natural extracts

This Example demonstrates the applicability of the MS based orphan bioassay method to rapidly determine ligands for orphan receptors, using a large set of natural extracts spiked with digoxin and structurally related molecules as model compounds. Unbiased determination of bioactive compounds using several software supported background substraction options is shown.

**Bioassay description** Affinity protein was added to a stream comprising the analytes. This mixture was then introduced in an open tubular knitted reaction coil, which allowed the affinity protein and sample compounds to interact for 30 s. After the affinity interaction, the mobile phase was led over a restricted access column in order to separate affinity complexes and non-bioactive compounds. The nature of the restricted access material enables the 'bulky' affinity complexes to pass the column unretained, whereas smaller unbound compounds are trapped inside the hydrophobic pores of the material.

After passing the first restricted access column the affinity complexes are dissociated by introducing a pH shock, i.e. adding a fairly acidic solution, which lowers the pH to 2. This dissociation reaction proceeds in only 10 s. Subsequently the mobile phase is led over a second restricted access column, which traps the bioactive compounds, whereas the affinity proteins are efficiently removed. Next the restricted access column is flushed with MilliQ water in order to remove excess of background ions and interfering compounds, after which the RA-column is switched into the desorption solution. Compounds trapped onto the RA-column elute and are introduced into the DECA mass spectrometer, which detects and characterizes the presence of bioactive compounds.

**Biochemical assay configuration** The biochemical assay part of the system consisted of five Knauer ministar LC-pumps. One LC-pump delivered the carrier phase, 10 mM TRIS and 10% DMSO pH 7.5, at a flow rate of 200 µl/min. A second LC-pump delivered 'plugs' of affinity protein, solved in carrier solution, at a maximum flow rate of 100 µl/min. The dissociation solution, consisting of 10% acetonitrile and 0.25%TFA, was pumped by a third LC-pump at a flow rate of 200 µl/min. In addition the second restricted access column, was flushed after each injection with MilliQ water for one minute at a flow rate of 1 ml/min. The fifth LC-pump delivered the desorption phase, 75% methanol and 2 mM ammonium acetate, and was operated at a flow rate of 100 µl/min which was directly introduced into the DECA mass spectrometer.

In order to separate affinity complexes from non-bioactive compounds present in the sample a 2*15 mm C18 ADS restricted access column was used. The second restricted access column, used to separate affinity protein from active compounds, consisted of similar material however differed in dimensions (2*5 mm).

The reaction coils used were knitted, open tubular, Teflon coils, with an internal diameter of 0.5 mm. The lengths of the reaction coils were chosen in such a way as to allow a 30 and 10 s reaction time for respectively the affinity interaction and the dissociation reaction.

**Specifications mass spectrometer** All measurements were performed on a Finnigan DECA ion trap mass spectrometer, which was equipped with an ESI probe. As the amount of information regarding potential interesting compounds is usually limited, the apparatus was tuned in such a way as to obtain acceptable sensitivity over a broad m/z range. Experiments were run using positive electrospray ionisation and a capillary temperature of 170 °C. The mobile phase, which originates from the biochemical assay part of the system, consisted of 75% of methanol and 2 mM ammonium acetate and was introduced at a flow rate of 100 µl/min.

**Data evaluation** Using Xcalibur 1.1, the operating software of the DECA mass spectrometer, the data was evaluated by performing several software operations on the collected dataset. First the raw datafile was corrected for the background by performing a simple background substraction routine on the samples. For this purpose several 'blank' samples were included when measuring a series of natural extracts.

After removing the background the corrected MS-spectrum was analysed for active compounds by generating reconstructed ion current spectra (RIC) for those m/z values, which still exhibited a relatively high intensity, meaning an intensity higher than the apparent noise level. By determining the intensity ratio of the m/z value between that observed in the blank sample and that measured in the natural extract, active compounds could be traced easily.

**Natural extract screening** Several natural extracts ranging from fungal to higher plant extracts were spiked with digoxin and cross-reactive compounds, *i*.*e*. gitoxin, 3, 12-diacetate digoxigenin, digoxigenin and gitoxigenin, in order to demonstrate the applicability of the method in discovering ligands for orphan receptors in complex extracts. The extracts were spiked with several concentrations of the bioactive compounds. The affinity protein used to simulate the interaction between orphan receptor and ligand was anti digoxigenin Fab, which exhibits reactivity for a range of digoxin related molecules.

### Results

**Natural extract screening** Natural extracts, dissolved in 100% DMSO, were tenfold diluted with carrier solution in order to reduce the DMSO concentration to an acceptable, bioassay compatible level. These extracts were then spiked with various concentrations of digoxin, 3, 12-diacetate digoxigenin, digoxigenin, gitoxin and gitoxigenin, i.e. compounds which not only differ from each other with respect to their affinity constant of the affinity interaction, but also in ionisation efficiency, meaning MS-detectability. Using these spiked samples the detection limits of the selected compounds were determined in a large set of natural extracts.

With respect to digoxin the minimum detection limit was determined to be approximately 6 pmol in higher plant extracts. Although some variation in detection limits was measured, due to matrix effects of the different sample types, the maximum detection limit was determined to be 13 pmol.

In addition, all other compounds could be detected in the natural extracts as well. Due to lower ionisation efficiencies with respect to digoxin, digoxigenin and gitoxigenin proved to be hardest compounds to detect. This is reflected by the detection limits of these compounds which were found to be 25-30 pmol.

**Data evaluation** The procedure used to evaluate the collected data, as described in the experimental section, proved to be highly suitable in making unbiased assignments of bioactive compounds. Using the background substraction option provided by Xcalibur, background ions could be efficiently removed, thus facilitating bioactive compound recognition.

In case of the bioactive compounds, used to test the system under real life conditions, the corresponding m/z ratios could easily be detected using this form of data evaluation. Additional peaks, which seemed interesting at a first glance, could easily be discarded as either spikes or incompletely removed background ions.

By comparing the MS-spectra of a natural extract obtained after simple sample cleanup and after orphan bioassay analysis, the shear power of the method to rapidly detect bioactive compounds for orphan receptors in complex matrixes, is clearly demonstrated. As the first spectrum shows a multitude of compounds still present after sample cleanup, the second spectrum shows a clean MS-spectrum displaying only the m/z values of digoxin as the principal ions.

## Claims

1. On-line detection method comprising the on-line coupling of the effluent of a fractionation step to a mass spectrometer, which effluent possibly contains analytes which method comprises the addition of a controlled amount of an affinity molecule to said effluent, whereby the affinity molecules bind analytes in the effluent, followed by a separation step using a restricted-access support, whereby the analyte-affinity molecule complex is permeated, while smaller molecules are retained in the restricted access column, followed by a suitable dissociation step to dissociate the analyte-affinity molecule complex, followed by a second separation step in which the dissociated analyte and affinity molecules are separated, followed by detection of the analyte using the mass spectrometer.

2. On-line detection method according to claim 1, in which the second separation step is carried out using a restricted-access support, in which the affinity molecule is retained, followed by elution of the analyte from the restricted-access support using a suitable carrier stream, and directing the eluted stream to the mass spectrometer.

3. On-line detection method according to claim 1, in which the second separation step is carried out using a hollow fiber support, whereby the analyte is permeated and the permeate is directed to the mass spectrometer.

4. On-line detection method according to any of the preceding claims, in which the dissociation step is a low pH shock, contacting with a high ionic strength solution, contacting with an organic solvent and/or contacting with a chaotropic reagent.

5. Method according to any of the preceding claims in which the fractionation step is a liquid chromatography separation or a capillary electrophoresis step, which is optionally followed by a separation step which removes the high molecular weight background.

6. Method according to claim 5, in which the liquid chromatography separation step is a HPLC, a reversed phase HPLC, a capillary electrophoresis, (CE), a capillary electrochromatography (CEC), an isoelectric focusing (IEF) or a micellar electrokinetic chromatography (MEKC) step.

7. Method according to any one of the preceding claims, in which the mass spectrometer is of the type chosen from the group consisting of electrospray ionization type, atmospheric pressure ionization type, quadrupole type, magnetic sector type, time-off-flight type, MS/MS, MSⁿ, FTMS type, ion trap type and combinations thereof.

8. Method according to any one of the preceding claims, in which the mass spectrometer is set to detect ions of a selected single m/z trace, selected multiple m/z traces, in scanning mode or any sequential mode.

9. The method of any of the preceding claims wherein the affinity molecule is an affinity protein.

10. The method of any of the preceding claims wherein the affinity molecule is an orphan receptor.

## Patentansprüche

1. Online-Nachweisverfahren, umfassend Online-Koppeln der Austrittsströmung einer Fraktionierungsstufe mit einem Massenspektrometer, wobei diese Austrittsströmung möglicherweise zu analysierende Stoffe enthält, bei welchem das Verfahren die Zugabe einer kontrollierten Menge eines Affinitätsmoleküls zu dieser Austrittsströmung umfaßt, wodurch die Affinitätsmoleküle zu analysierenden Stoffe in der Austrittsströmung binden, gefolgt von einer Trennstufe unter Verwendung eines Trägers mit eingeschränktem Zugang, wodurch der Komplex aus zu analysierendem Stoff-Affinitätsmolekül permeiert wird, während kleinere Moleküle in der Säule mit eingeschränktem Zugang zurückgehalten werden, gefolgt von einer geeigneten Dissoziationsstufe zum Dissoziieren des Komplexes aus zu analysierendem Stoff-Affinitätsmolekül, gefolgt von einer zweiten Trennstufe, in welcher der dissoziierte zu analysierende Stoff und die Affinitätsmoleküle getrennt werden, gefolgt vom Nachweis des zu analysierenden Stoffes unter Anwendung des Massenspektrometers.

2. Online-Nachweisverfahren entsprechend Anspruch 1, bei welchem die zweite Trennstufe unter Verwendung eines Trägers mit eingeschränktem Zugang durchgeführt wird, wobei das Affinitätsmolekül zurückgehalten wird, gefolgt von Elution des zu analysierenden Stoffes aus dem Träger mit eingeschränktem Zugang unter Verwendung einer geeigneten Trägerströmung, sowie Leiten der eluierten Strömung zu dem Massenspektrometer.

3. Online-Nachweisverfahren entsprechend Anspruch 1, bei welchem die zweite Trennstufe unter Verwendung eines Hohlfaserträgers durchgeführt wird, wodurch der zu analysierende Stoff permeiert wird, und das Permeat zu dem Massenspektrometer geleitet wird.

4. Online-Nachweisverfahren entsprechend irgendeinem der vorhergehenden Ansprüche, bei welchem die Dissoziationsstufe eine Schock mit niedrigem pH ist durch Kontaktieren mit einer Lösung mit hoher Ionenstärke, durch Kontaktieren mit einem organischen Lösungsmittel und/oder Kontaktieren mit einem chaotropischen Mittel.

5. Verfahren entsprechend irgendeinem der vorhergehenden Ansprüche, bei welchem die Fraktionierungsstufe eine Stufe durch Trennung mittels Flüssigkeitschromatographie oder mittels Kapillarelektrophorese ist, welche wahlweise von einer Trennstufe gefolgt wird, welche den Untergrund mit hohem Molekulargewicht entfernt.

6. Verfahren entsprechend Anspruch 5, bei welchem die Trennstufe mittels Flüssigkeitschromatographie eine Stufe mittels HPLC, mittels HPLC mit umgekehrter Phase, mittels Kapillarelektrophorese (CE), mittels Kapillarelektrochromatographie (CEC), mittels isoelektrischer Fokussierung (IEF) oder mittels micellarer elektrokinetischer Chromatographie ist.

7. Verfahren entsprechend irgendeinem der vorhergehenden Ansprüche, bei welchem das Massenspektrometer von dem Typ ist, der aus der Gruppe ausgewählt ist, die aus dem Elektrosprayionisationstyp, Ionisationstyp bei atmosphärischem Druck, Quadrupoltyp, Magnetsektortyp, Flugzeittyp, MS/MS-, MSⁿ-, FTMS-Typ, Ionenfallentyp und Kombinationen hiervon besteht.

8. Verfahren entsprechend irgendeinem der vorhergehenden Ansprüche, bei welchem das Massenspektrometer zum Nachweis einer einzelnen m/s-Spur, ausgewählten m/z-Spuren, in Scanmodus oder einem beliebigen Sequenzmodus eingestellt ist.

9. Verfahren entsprechend irgendeinem der vorhergehenden Ansprüche, bei welchem das Affinitätsmolekül ein Affinitätsprotein ist.

10. Verfahren entsprechend irgendeinem der vorhergehenden Ansprüche, bei welchem das Affinitätsmolekül ein Orphanrezeptor ist.

## Revendications

1. Procédé de détection en ligne comprenant le couplage en ligne de l'effluent d'une étape de fractionnement à un spectromètre de masse, cet effluent contenant éventuellement des analytes, lequel procédé comprend l'addition d'une quantité réglée d'une molécule d'affinité audit effluent, les molécules d'affinité liant les analytes dans l'effluent, suivie d'une étape de séparation utilisant un support à accès limité, le complexe d'analyte-molécule d'affinité passant par perméation tandis que les molécules plus petites sont retenues dans la colonne à accès limité, suivie d'une étape de dissociation appropriée pour dissocier le complexe d'analyte-molécule d'affinité, suivie d'une seconde étape de séparation dans laquelle l'analyte et les molécules d'affinité dissociés sont séparés, suivie d'une détection de l'analyte en utilisant le spectromètre de masse.

2. Procédé de détection en ligne selon la revendication 1, dans lequel la seconde étape de séparation est effectuée en utilisant un support à accès limité, dans lequel la molécule d'affinité est retenue, puis en éluant l'analyte à partir du support à accès limité en utilisant un courant porteur approprié, et en envoyant le courant élué au spectromètre de masse.

3. Procédé de détection en ligne selon la revendication 1, dans lequel la seconde étape de séparation est effectuée en utilisant un support en fibre creuse, l'analyte passant par perméation et le perméat étant envoyé au spectromètre de masse.

4. Procédé de détection en ligne selon l'une quelconque des revendications précédentes, dans lequel l'étape de dissociation est un choc à pH bas, une mise en contact avec une solution à grande force ionique, une mise en contact avec un solvant organique et/ou une mise en contact avec un réactif chaotropique.

5. Procédé selon l'une quelconque des revendications précédentes, dans lequel l'étape de fractionnement est une séparation par chromatographie en phase liquide ou une étape d'électrophorèse capillaire, qui est facultativement suivie d'une étape de séparation qui élimine la composante de fond de haut poids moléculaire.

6. Procédé selon la revendication 5, dans lequel l'étape de séparation par chromatographie en phase liquide est une CLHP, une CLHP en phase inversée, une électrophorèse capillaire (EC), une électrochromatographie capillaire (ECC), une isoélectrofocalisation (IEF) ou une étape de chromatographie électrocinétique micellaire (CECM).

7. Procédé selon l'une quelconque des revendications précédentes, dans lequel le spectromètre de masse est du type choisi dans le groupe formé par le type à ionisation par électronébulisation, le type à ionisation à la pression atmosphérique, le type quadripolaire, le type à secteurs magnétiques, le type à temps de vol, le type MS/MS, MSₙ, FTMS, le type à piège à ions et leurs combinaisons.

8. Procédé selon l'une quelconque des revendications précédentes, dans lequel le spectromètre de masse est réglé pour détecter les ions d'une seule trace m/z sélectionnée, de multiples traces m/z sélectionnées, en mode de balayage ou en tout mode séquentiel.

9. Le procédé de l'une quelconque des revendications précédentes, dans lequel la molécule d'affinité est une protéine d'affinité.

10. Le procédé de l'une quelconque des revendications précédentes, dans lequel la molécule d'affinité est un récepteur orphelin.
